# FASCICULE DE BREVET EUROPEEN

(11) **EP 1 521 608 B1**
(45) Date de publication et mention de la délivrance du brevet: **15.12.2010**
(21) Numéro de dépôt: 03756494.5
(22) Date de dépôt: 10.07.2003
(51) Int. Cl.: A61M 5/34, A61F 9/007

(54) **ACCESSOIRE POUR SERINGUE**
ZUBEHÖRTEIL FÜR SPRITZEN
SYRINGE ACCESSORY

(30) Priorité: 12.07.2002 FR 0208845
(43) Date de publication de la demande: 13.04.2005
(73) Titulaire: Becton Dickinson France, 38800 Le Pont de Claix (FR)
(72) Inventeur: PEROT, Frederic, F-38760 Saint Paul De Varces (FR); VEDRINE, Lionel, Palo Alto, California 94306 (US)
(74) Mandataire: Brédeville, Odile Marie
(86) Numéro de dépôt international: PCT/FR2003/002179
(87) Numéro de publication internationale: WO 2004/007006

(56) Documents cités:
- EP-A- 0 845 275
- WO-A-94/13347
- FR-A- 574 431
- FR-A- 963 751
- FR-A- 2 783 433
- US-A- 4 904 244
- US-A- 5 876 379
- US-A- 5 925 032

## Description

La présente invention concerne un accessoire pour seringue. Dans certains cas, une injection réalisée au moyen d'une seringue a pour effet de générer une contrainte longitudinale sur l'aiguille lors de l'injection. Cette contrainte risque de conduire à une séparation de l'aiguille, et/ou de son embout de connexion, par rapport au corps de seringue au cours de l'injection. Tel est notamment le cas lorsqu'il est nécessaire d'injecter un produit visqueux.

Une connexion par vissage, dite "luer-lock", remédie en partie à ce problème, mais ne peut pour autant interdire un dévissage de l'aiguille sous ladite contrainte. Un tel dévissage, même partiel, peut provoquer des lésions aux tissus environnants, tant du fait de l'injection de produit que du mouvement intempestif de l'aiguille, en particulier lorsque celle-ci est courbe ou coudée. Ce problème se pose particulièrement dans le domaine de la chirurgie oculaire, dans lequel des produits visqueux et des aiguilles courbes sont utilisés.

Il existe un accessoire pour seringue notamment employé en chirurgie oculaire, comprenant un élément tubulaire, un écrou et un anneau de maintien. L'élément tubulaire est destiné à recevoir intérieurement un corps de seringue et forme un filetage externe distal. L'écrou comprend un fond percé d'un trou et est destiné à être engagé sur l'aiguille grâce à ce trou, puis à être vissé sur le filet de l'élément tubulaire jusqu'à ce que son fond prenne appui contre l'embout de connexion de l'aiguille, permettant ainsi la rétention de aiguille par rapport au corps de seringue. L'anneau de maintien assure quant à lui le maintien du corps de seringue dans l'élément tubulaire.

Cet accessoire a pour inconvénient de rendre possible un endommagement ou une contamination de l'aiguille lors de la mise en place de l'écrou. De plus, il a une structure relativement complexe et implique un préchargement de la seringue dans l'élément tubulaire, relativement contraignant à réaliser, ce qui n'est pas sans incidences sur le coût de sa fabrication et de son utilisation.

Le document US 5,925,032 décrit un accessoire pour seringue, comprenant une partie cylindrique au travers de laquelle l'aiguille doit être enfilée, et deux parties en semi-tubes se refermant sur l'aiguille.

L'invention a pour but de remédier à ces inconvénients.

L'accessoire qu'elle concerne comprend, de manière connue en soi, un corps, des premiers moyens de maintien prenant appui contre l'aiguille de la seringue ou contre l'embout de connexion de cette aiguille à cette seringue, et des deuxièmes moyens de maintien prenant appui contre le corps de seringue, ces premiers et seconds moyens de maintien permettant de maintenir l'aiguille sur le corps de seringue lorsqu'une contrainte dans le sens longitudinal de la seringue est exercée sur l'aiguille lors de l'injection, tendant à séparer l'aiguille du corps de seringue.

Selon l'invention,
- le corps de l'accessoire est de forme sensiblement hémi-tubulaire ;
- lesdits premiers moyens de maintien comprennent une paroi transversale distale reliée à une extrémité dudit corps, percée d'un trou pour le passage de l'aiguille au travers d'elle, et
- lesdits deuxièmes moyens de maintien comprennent une zone d'appui contre laquelle le corps de seringue est destiné à venir en appui,
   la distance entre ladite paroi transversale distale et ladite zone d'appui étant telle que l'embout de connexion de l'aiguille au corps de seringue est maintenu en appui contre ladite paroi transversale distale lorsque le corps de seringue est en appui contre ladite zone d'appui.

Le corps de l'accessoire permet, de par sa forme, un engagement facile et rapide de la seringue en lui, et ladite paroi transversale distale élimine l'engagement d'un écrou sur l'aiguille, donc le risque de détérioration ou de contamination de cette aiguille au cours de cet engagement. De plus et surtout, l'appui de l'embout de connexion contre ladite paroi transversale distale génère des frottements entre cet embout et cette paroi, à même d'empêcher le pivotement de l'aiguille, lorsque celle-ci est reliée au corps de seringue par une connexion vissée, notamment de type "luer-lock", sous ladite contrainte longitudinale.

Le corps de l'accessoire est constitué d'un demi-tube.

De préférence, l'accessoire comprend au moins un moyen permettant d'assurer un calage de l'embout de l'aiguille en rotation par rapport au corps de l'accessoire.

Ce calage en rotation permet de fixer en rotation l'embout de l'aiguille lorsque ce dernier est relié au corps de seringue par une connexion vissée, notamment de type "luer-lock".

Ce moyen de calage peut notamment se présenter sous forme d'au moins un cran faisant saillie de ladite paroi transversale distale et/ou d'un rebord attenant à celle-ci, qui est destiné à coopérer avec au moins une nervure que comprend fréquemment l'embout de connexion de l'aiguille au corps de seringue.

Lorsque l'accessoire comprend plusieurs de ces crans, les crans peuvent notamment être disposés autour du trou que comprend ladite paroi transversale distale pour permettre le passage de l'aiguille, selon une direction radiale par rapport à ce trou.

Alternativement à ces moyens de calage, ou en complément de ceux-ci, l'embout peut présenter une forme plus ou moins conique ou cylindro-conique et être destiné à être reçu dans ledit trou de ladite paroi transversale avec coincement.

L'accessoire est avantageusement réalisé en une seule pièce, notamment par moulage d'une matière synthétique.

Il peut ainsi être fabriqué à un prix de revient réduit.

Avantageusement, ledit corps de l'accessoire est réalisé en deux parties, dont une comprend ladite paroi transversale distale et l'autre ladite zone d'appui, ces deux parties étant reliées l'une à l'autre par une zone élastique, étirable dans la direction longitudinale de l'accessoire.

Cette zone élastique permet l'adaptation de l'accessoire à des corps de seringues de différentes longueurs ou aux tolérances de fabrication ou d'assemblage que peuvent présenter des corps de seringue de longueur standard assemblés avec une aiguille.

Ladite zone élastique peut notamment comprendre au moins une portion courbe, ajourée, ondulée ou hélicoïdale, reliant lesdites parties du corps de l'accessoire.

Selon l'invention, le trou précité que comprend ladite paroi transversale distale pour le passage de l'aiguille débouche sur l'extérieur de cette paroi par l'intermédiaire d'au moins une fente, cette fente permettant un engagement latéral de l'aiguille dans le trou.

Cet engagement est ainsi facile et à même de préserver l'intégrité de l'extrémité distale de l'aiguille. Il peut être réalisé simultanément à l'engagement du corps de seringue dans ledit corps de l'accessoire, par le même geste.

Ladite zone d'appui de l'extrémité proximale du corps de seringue peut être conformée pour former une butée autorisant le coulissement de la tige de piston, mais se trouvant sur la course de recul du piston de la seringue ou d'une partie de la tige de piston.

L'accessoire empêche ainsi la sortie du piston hors du corps de seringue.

Ladite zone d'appui peut être délimitée par au moins une paroi transversale proximale qu'elle comprend. Selon l'invention, dans ce cas, l'accessoire comprend deux parois transversales proximales, sensiblement parallèles, décalées dans le sens longitudinal de l'accessoire, qui délimitent entre elles un logement de réception de la collerette proximale ou des pattes latérales proximales que comprend éventuellement le corps de la seringue.

Ce logement peut être ajusté à cette collerette proximale ou à ces pattes latérales proximales de manière à assurer le montage de l'accessoire sur la seringue par frottements.

Selon l'invention ce logement s'ouvre latéralement selon la même direction que celle selon laquelle ledit trou de passage de l'aiguille communique avec l'extérieur de l'accessoire par ladite fente.

L'engagement de la seringue dans l'accessoire est ainsi facilité.

Alternativement ou cumulativement, le montage de l'accessoire sur la seringue peut être assuré par encliquetage de la seringue dans l'accessoire, ce dernier comprenant des moyens à cet effet.

L'accessoire peut en outre comprendre une paroi de liaison formant, au niveau de sa face tournée vers la paroi transversale distale destinée à recevoir l'embout de l'aiguille, des surfaces latérales, de part et d'autre du corps de l'accessoire, ces surfaces latérales étant destinées à recevoir les doigts de l'utilisateur et étant conformées de façon ergonomique à cet effet.

Le corps de l'accessoire peut présenter deux bords longitudinaux délimitant le logement de réception du corps de seringue, ces bords ayant des formes allant en s'amincissant en direction de leurs bords libres afin de faciliter l'utilisation de l'accessoire en relation avec la seringue.

Pour sa bonne compréhension, l'invention est à nouveau décrite ci-dessous en référence au dessin schématique annexé, représentant, à titre d'exemple non limitatif, une forme de réalisation préférée de l'accessoire pour seringue qu'elle concerne.
La figure 1 en est une vue en perspective ;
la figure 2 en est une vue en perspective, après mise en place sur lui d'une seringue ;
la figure 3 en est une vue partielle, en perspective et à échelle agrandie, et
les figures 4 à 6 sont des vues de côté de différentes variantes de réalisation de l'accessoire.

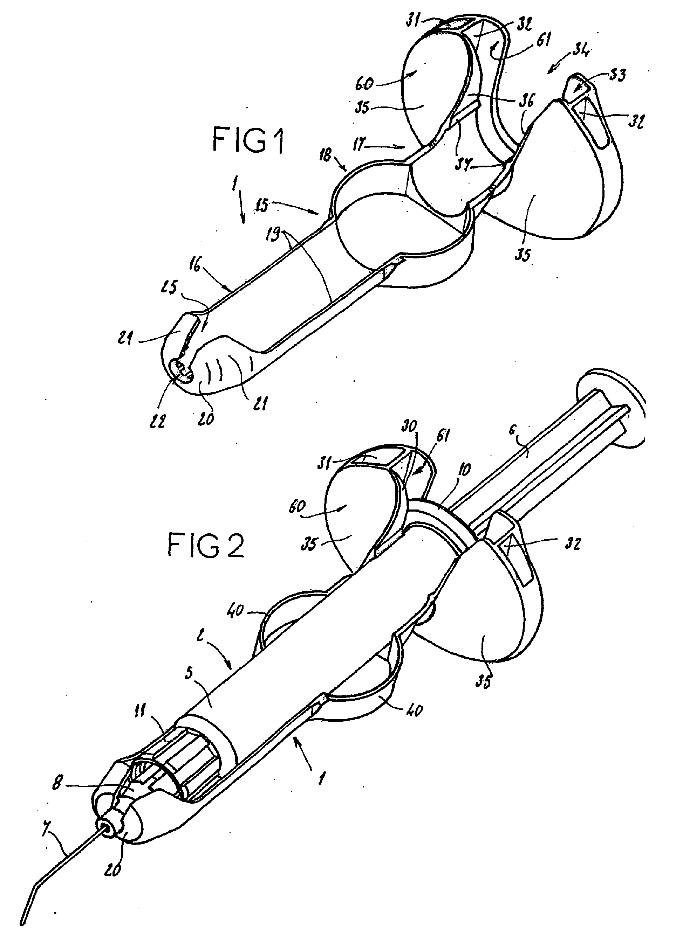

Les parties ou éléments de l'accessoire montré sur les figures 1 à 3, qui se retrouvent de manière identique ou similaire sur les différentes variantes de réalisation, seront repérés par les mêmes références numériques et ne seront pas à nouveau décrits.

La figure 1 représente un accessoire 1 pour seringue et la figure 2 représente une seringue 2 mise en place dans cet accessoire 1.

Cette seringue 2, notamment destinée à être utilisée dans le domaine de la chirurgie oculaire, est de type classique, comprenant un corps de seringue 5, un piston actionné par une tige 6, une aiguille 7et un embout 8 de connexion de l'aiguille 7 au corps de seringue 5.

Ce dernier comprend une collerette 10 proximale et, du côté opposé à cette collerette 10, un embout distal, non visible sur les figures, de connexion de l'embout 8 de l'aiguille 7.

L'aiguille 7 est coudée dans l'exemple représenté, comme cela peut être pratiqué dans le domaine de la chirurgie oculaire.

L'embout de connexion 8 présente une forme plus ou moins conique ou cylindro-conique et comprend des nervures (non visibles sur la figure 2), faisant saillie de sa paroi à son niveau distal. Dans l'exemple représenté, il coopère avec un écrou 11, de type "luer-lock", permettant une connexion de l'embout 8 au corps de seringue 5 par vissage de l'embout 8 dans cet écrou 11.

En référence à la figure 1, il apparaît que l'accessoire 1 comprend un corps 15 en deux parties 16, 17, reliées l'une à l'autre par une zone élastique 18, élastiquement étirable.

Le corps 15 présente une forme hémi-cylindrique et est conformé pour envelopper sensiblement la moitié du corps de seringue 5. Il présente deux bords longitudinaux 19 délimitant le logement de réception du corps de seringue 5, ces bords 19 ayant des formes allant en s'amincissant en direction de leurs bords libres afin de faciliter l'utilisation de l'accessoire avec la seringue, par l'utilisateur (chirurgien).

La partie 16 présente une paroi transversale distale 20, bordée par un rebord latéral semi-périphérique 21, dans laquelle est aménagé un trou 22 pour le passage de l'aiguille 7. Dans l'exemple représenté, ce trou 22 est dimensionné pour recevoir l'extrémité distale de l'embout 8 avec coincement compte tenu de la forme plus ou moins conique de cet embout 8.

Dans l'exemple représenté, le trou 22 communique avec l'extérieur de l'accessoire 1 par une fente 25 permettant l'engagement de l'aiguille 7 à travers elle, latéralement par rapport à l'accessoire 1, lors de la mise en place de la seringue 2 dans l'accessoire 1.

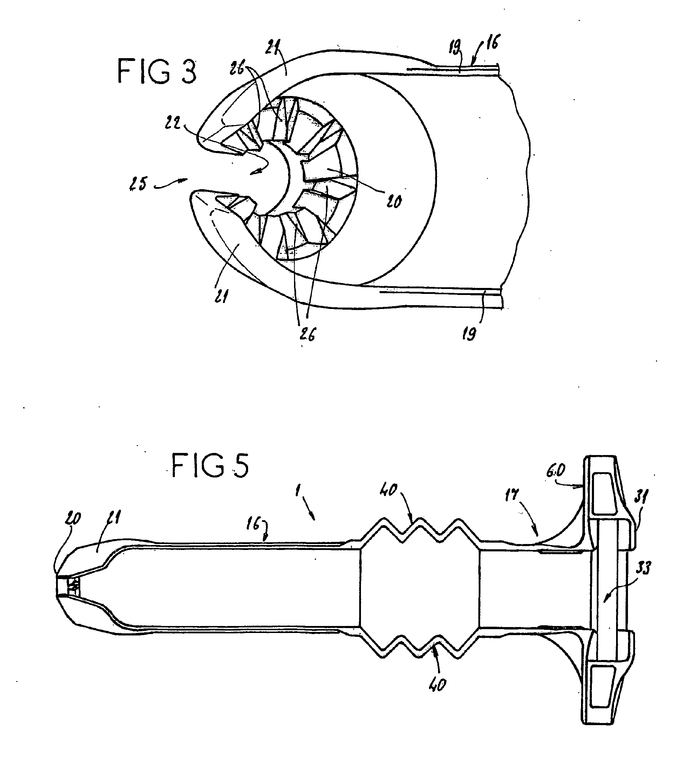

En outre, comme le montre la figure 3, la paroi transversale distale 20 et le rebord 21 comprennent des crans 26 faisant saillie de sa face proximale, disposés autour du trou 22 selon une orientation radiale par rapport à ce trou 22. Ces crans 26 sont destinés à coopérer avec les nervures précitées que comprend l'embout 8.

La partie 17 comprend deux parois transversales proximales, sensiblement parallèles 30, 31, décalées dans le sens longitudinal de l'accessoire 1, et éventuellement deux cloisons 32 de rigidification. Les parois 30, 31 forment ensemble une zone d'appui 61 et délimitent entre elles un logement 33 destiné à recevoir la collerette 10, et présentent deux encoches 34 recevant respectivement la partie tubulaire du corps de seringue 5 et la tige de piston 6, comme le montre la figure 2. Ces encoches 34 sont orientées sensiblement dans la même direction que la fente 25.

Au niveau des entrées de ces encoches 34, les parois 30, 31 sont évasées, afin de faciliter l'engagement de la partie proximale de la seringue 2, de la tige de piston 6, et en particulier de la collerette 10, dans le logement 33. Dans le cas de l'utilisation d'une « carpule », au lieu d'une seringue, ces mêmes parois transversales proximales 30 et 31 facilitent l'entrée de seulement l'extrémité proximale du corps de la "carpule" et de la tige de piston.

Une paroi 60 de liaison peut présenter en outre des surfaces latérales 35, de part et d'autre du corps 15 de l'accessoire 1. Ces surfaces 35 sont destinées à recevoir les doigts de l'utilisateur et sont conformées de façon ergonomique à cet effet.

La partie 17 comprend également deux parois longitudinales parallèles 36, attenantes à la paroi 30 de liaison 60, qui prolongent les bords du corps 15. Au moins une de ces parois comporte au moins une nervure 37 d'encliquetage du corps de seringue 5 dans ce corps 15.

La zone élastique 18 comprend deux portions courbes 40 dont les faces convexes sont tournées vers l'extérieur de l'accessoire 1. Ces portions courbes 40 permettent l'adaptation de l'accessoire 1, soit à des corps de seringues 5 de différentes longueurs, soit aux tolérances de fabrication que peuvent présenter des corps de seringue 5 de longueur standard, et l'assemblage d'un ensemble aiguille 7-embout 8 à un corps de seringue 5 placé dans l'accessoire 1.

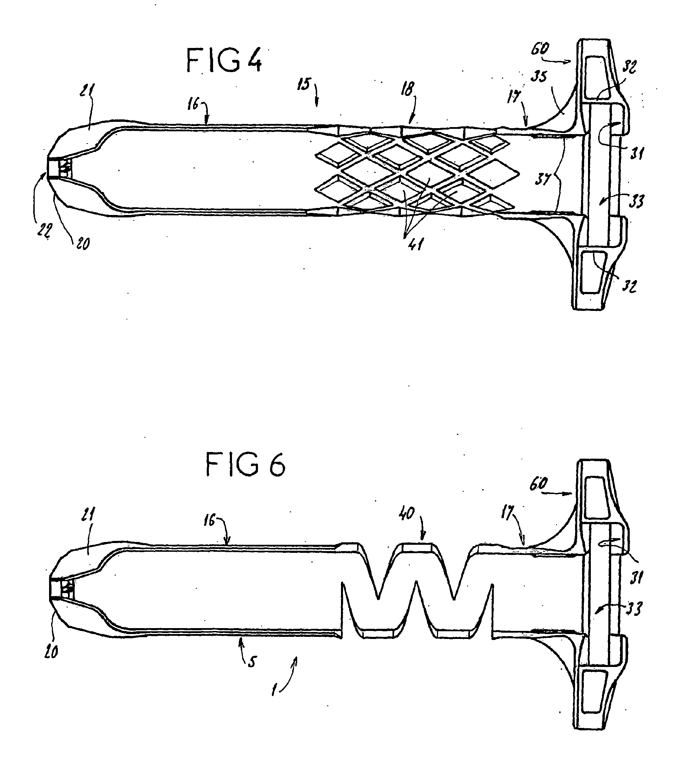

Les figures 4 à 6 montrent respectivement que la zone élastique 18 peut être formée par des ajours 41 aménagés dans le corps 15, ou qu'elle peut présenter des portions 40 ondulées ou une portion 40 hémi-hélicoïdale.

Il apparaît de ce qui précède que l'invention apporte une amélioration déterminante à la technique antérieure, en fournissant un accessoire pour seringue n'exposant pas l'aiguille à un risque d'endommagement ou de contamination lors de son utilisation, et ayant une structure simple et peu onéreuse à fabriquer, qui n'implique pas un préchargement de la seringue dans l'accessoire par le fabricant ou un tiers.

Il va de soi que l'invention n'est pas limitée à la forme de réalisation décrite ci dessus à titre d'exemple mais qu'elle en embrasse au contraire toutes les variantes de réalisation couvertes par les revendications ci-annexées ; ainsi, le produit contenu dans la seringue peut ne pas être visqueux, l'aiguille peut ne pas être coudée, la connexion de l'aiguille peut ne pas être de type "luer-lock", l'accessoire peut recevoir des seringues aussi bien vides que préremplies, le trou 22 peut communiquer avec l'extérieur de l'accessoire 1 par une ou plusieurs fentes 25 ; le terme seringue doit être compris de manière large, comme incluant des contenants similaires couramment dénommés "carpules" ou "cartridge", c'est-à-dire à corps 5 sans collerette 10, ou avec seulement un rebord, comportant un piston de poussée du liquide contenu dans ledit corps.

Le domaine d'application de la présente invention n'est pas limité à celui de la chirurgie ophtalmique, mais peut être étendu à beaucoup d'autres domaines médicaux, comme celui des soins dentaires avec utilisation d'un anesthésique, etc.

## Revendications

1. Accessoire (1) pour seringue (2), comprenant un corps (15), des premiers moyens de maintien (20) permettant l'appui contre l'aiguille (7) de la seringue ou contre l'embout (8) de connexion de cette aiguille (7) à cette seringue (2), et des deuxièmes moyens de maintien (61) permettant l'appui contre le corps de seringue (5), ces premiers et seconds moyens de maintien (20 ; 61) permettant de maintenir l'aiguille (7) sur le corps de seringue (5) lorsqu'une contrainte dans le sens longitudinal de la seringue (2) est exercée sur l'aiguille. (7) lors de l'injection, tendant à séparer l'aiguille (7) du corps de seringue (5),
- ledit corps (15) étant de forme sensiblement hémi-tubulaire ;
- lesdits premiers moyens de maintien comprennant une paroi transversale distale (20) reliée à une extrémité dudit corps (15), percée d'un trou (22) pour le passage de l'aiguille (7) au travers d'elle,
- lesdits deuxièmes moyens de maintien comprennant une zone d'appui (61) contre laquelle l'extrémité proximale du corps de seringue (5) est destinée à venir en appui,
la distance entre ladite paroi transversale distale (20) et ladite zone d'appui (61) étant telle que, lors de l'utilisation, l'embout (8) de connexion de l'aiguille (7) au corps de seringue (5) est maintenu en appui contre ladite paroi transversale distale (20) lorsque le corps de seringue (5) est en appui contre ladite zone d'appui (61),
ledit corps (15) est constitué d'un demi-tube,
le trou (22) que comprend ladite paroi transversale distale (20) pour le passage de l'aiguille (7) débouche sur l'extérieur de cette paroi par l'intermédiaire d'au moins une fente (25), cette fente (25) permettant un engagement latéral de l'aiguille (7) dans le trou (22), et
deux parois transversales proximales sensiblement parallèles (30, 31), décalées dans le sens longitudinal, qui délimitent entre elles un logement (33) de réception de la collerette proximale (10) ou des pattes latérales proximales que comprend éventuellement le corps (5) de la seringue (2), accessoire (1) **caractérisé : en ce que** ledit logement (33) s'ouvrant latéralement selon sensiblement la même direction que celle selon laquelle ledit trou (22) de passage de l'aiguille (7) communique avec l'extérieur de l'accessoire (1) par ladite fente (25).

2. Accessoire (1) selon la revendication 1, **caractérisé en ce qu'**il comprend au moins un moyen (26) permettant d'assurer un calage en rotation de l'embout de connexion (8) par rapport au corps (15) de l'accessoire (1).

3. Accessoire (1) selon la revendication 2, **caractérisé en ce que** ledit moyen de calage se présente sous forme d'au moins un cran (26) faisant saillie de ladite paroi transversale distale (20) et/ou d'un rebord (21) attenant à celle-ci.

4. Accessoire (1) selon la revendication 3, **caractérisé en ce qu'**il comprend plusieurs crans (26) et **en ce que** les crans (26) sont disposés autour du trou (22) que comprend ladite paroi transversale distale (20) pour permettre le passage de l'aiguille (7), selon une direction radiale par rapport à ce trou (22).

5. Accessoire (1) selon l'une des revendications 1 à 4, **caractérisé en ce que** l'embout (8) de connexion de l'aiguille (7) au corps de seringue (5) présente une forme plus ou moins conique, ou cylindro-conique, et est destiné à être reçu dans ledit trou (22) de ladite paroi transversale distale (20) avec coincement.

6. Accessoire (1) selon l'une des revendications 1 à 5, **caractérisé en ce qu'**il est réalisé en une seule pièce, notamment par moulage d'une matière synthétique.

7. Accessoire (1) selon l'une des revendications 1 à 6, **caractérisé en ce que** son corps (15) est réalisé en deux parties (16, 17), dont une comprend ladite paroi transversale (20) et l'autre ladite zone d'appui (61), ces deux parties (16, 17) étant reliées l'une à l'autre par une zone élastique (18), étirable dans la direction longitudinale de l'accessoire (1).

8. Accessoire (1) selon la revendication 7 **caractérisé en ce que** ladite zone élastique (18) comprend au moins une portion (40) courbe, ajourée, ondulée ou hélicoïdale, reliant lesdites parties (16, 17) du corps (15) de l'accessoire (1).

9. Accessoire (1) selon l'une des revendications 1 à 8, **caractérisé en ce que** ladite zone d'appui ( 61) est conformée pour former une butée autorisant le coulissement de la tige de piston (6) mais se trouvant sur la course de recul du piston de la seringue (2) ou d'une partie de la tige de piston.

10. Accessoire (1) selon l'une des revendications 1 à 9, **caractérisé en ce que** ladite zone d'appui (61) est délimitée par au moins une paroi transversale proximale (31) qu'elle comprend.

11. Accessoire (1) selon la revendication 1, **caractérisé en ce que** ledit logement (33) est ajusté à ladite collerette proximale (10) ou auxdites pattes latérales proximales.

12. Accessoire (1) selon l'une des revendications 1 à 11, **caractérisé en ce qu'**il comprend des moyens d'encliquetage de la seringue en lui.

13. Accessoire (1) selon l'une des revendications 1 à 12, **caractérisé en ce qu'**il comprend une paroi de liaison (60) formant, au niveau de sa face tournée vers la paroi transversale (20) destinée à recevoir l'embout (8) de connexion de l'aiguille (7), des surfaces latérales (35), de part et d'autre du corps (15) de l'accessoire (1), ces surfaces latérales (35) étant destinées à recevoir les doigts de l'utilisateur et étant conformées de façon ergonomique à cet effet.

14. Accessoire (1) selon l'une des revendications 1 à 13, **caractérisé en ce que** son corps (15) présente deux bords longitudinaux (19) délimitant le logement de réception du corps de seringue (5), ces bords (19) ayant des formes allant en s'amincissant en direction de leurs bords libres.

15. Accessoire (1) selon l'une des revendications 1 à 14, **caractérisé en ce qu'**il est adapté à un contenant du type « carpule » ou « cartridge ».

## Claims

1. An accessory (1) for a syringe (2), comprising a body (15), first holding means bearing against the needle (7) of the syringe or against the adapter (8) that connects this needle (7) to this syringe (2), and second holding means (61) bearing against the syringe body (5), these first and second holding means (20, 61) allowing the needle (7) to be held on the syringe body (5) when stress is exerted on the needle (7) in the longitudinal direction of the syringe (2) at the time of injection, with a tendency to separate the needle (7) from the syringe body (5) ;
wherein:
- said body (15) is of roughly semi-tubular shape;
- said first holding means comprise a distal transverse wall (20) connected to one end of said body (15) and pierced with a hole (22) for the passage of the needle (7) through it, and
- said second holding means comprise a bearing zone (61) against which the proximal end of the syringe body (5) is intended to bear,
the distance between said distal transverse wall (20) and said bearing zone (61) being such that the adapter (8) connecting the needle (7) to the syringe body (5) is kept bearing against said distal transverse wall (20) when the syringe body (5) bears against said bearing zone (61),
the body (15) consists in a semi-tube,
the hole (22) that said distal transverse wall (20) comprises for the passage of the needle (7) opens to the outside of this wall via at least one slot (25), this slot (25) allowing the needle (7) to be engaged in the hole (22) laterally,
two roughly parallel proximal transverse walls (30, 31) offset in the longitudinal direction and which between them delimit a housing (33) for accommodating the proximal flange (10) or proximal lateral tabs that the body (5) of the syringe (2) might have,
the accessory being **characterized in that** said housing (33) opens laterally in roughly the same direction as the direction in which said hole (22) for the passage of the needle (7) communicates with the outside of the accessory (1) via said slot (25).

2. The accessory (1) as claimed in claim 1, and which comprises at least one means (26) allowing the connecting adapter (8) to be prevented from rotating with respect to the body (15) of the accessory.

3. The accessory as claimed in claim 2, wherein said rotation-preventing means is in the form of at least one tooth (26) projecting from said distal transverse wall (20) and/or of a rim (21) contiguous therewith.

4. The accessory (1) as claimed in claim 3, and which comprises several teeth (26) and wherein the teeth (26) are arranged around the hole (22) that said distal transverse wall (20) comprises for allowing the passage of the needle (7), in a radial direction with respect to this hole (22).

5. The accessory (1) as claimed in one of claims 1 to 4, wherein the adapter (8) connecting the needle (7) to the syringe body (5) has a more or less conical or cylindro-conical shape and is intended to be jammed into said hole (22) in the distal transverse wall (20).

6. The accessory (1) as claimed in one of claims 1 to 5, and which it is made as a single piece, particularly by molding in a synthetic material.

7. The accessory (1) as claimed in one of claims 1 to 6, and of which the body (15) is made in two parts (16, 17), one of which comprises said transverse wall (20) and the other of which comprises said bearing zone (61), these two parts (16, 17) being connected to one another by an elastic zone (18) that can be stretched in the longitudinal direction of the accessory (1).

8. The accessory (1) as claimed in claim 7, wherein said elastic zone (18) comprises at least one curved, perforated, undulating or helicoid portion (40) connecting said parts (16, 17) of the body of the accessory (1).

9. The accessory (1) as claimed in one of claims 1 to 8, wherein said bearing zone (61) is shaped to form a stop allowing the piston plunger (6) to slide but lying in the return path of the piston of the syringe (2) or part of the piston plunger.

10. The accessory (1) as claimed in one of claims 1 to 9, wherein said bearing zone (61) is delimited by at least one proximal transverse wall (31) that it has.

11. The accessory (1) as claimed in claim 1, wherein said housing (33) is tailored to said proximal flange (10) or said proximal lateral tabs.

12. The accessory (1) as claimed in one of claims 1 to 11, and which comprises means for snap-fastening the syringe into it.

13. The accessory (1) as claimed in one of claims 1 to 12, and which comprises a connecting wall (60) which, at its face facing toward the transverse wall intended to accommodate the adapter (8) for connecting the needle, forms lateral surfaces on each side of the body (15) of the accessory (1), these lateral surfaces (35) being intended to accommodate the user's fingers and being shaped ergonomically for that purpose.

14. The accessory (1) as claimed in one of claims 1 to 13, and of which the body (15) has two longitudinal edges (19) delimiting the housing that accommodates the syringe body (5), these edges (19) having shapes which taper toward their free edges.

15. The accessory (1) as claimed in one of claims 1 to 14, and which is designed for a container of the "carpule" or "cartridge" type.

## Patentansprüche

1. Zubehörteil (1) für eine Spritze (2), das einen Körper (15), erste Haltemittel (20), die ein Abstützen gegen die Nadel (7) der Spritze oder gegen das Anschlussstück (8) zur Verbindung der Nadel (7) mit der Spritze (2) ermöglichen, und zweite Haltemittel (61) aufweist, die ein Abstützen gegen den Körper der Spritze (5) ermöglichen, wobei die ersten und zweiten Haltemittel (20; 61) es ermöglichen, die Nadel (7) am Körper der Spritze (5) zu halten, wenn während der Injektion auf die Nadel (7) Belastungen in Längsrichtung der Spritze (2) ausgeübt werden, die dazu tendieren, die Nadel (7) vom Körper der Spritze (5) abzuziehen,
- wobei der Körper (15) eine im Wesentlichen halbröhrenförmige Form aufweist;
- wobei die ersten Haltemittel eine distale Querwand (20) aufweisen, die an einem Ende des Körpers (15) angeordnet ist und die durch ein Loch (22) zum Durchgang der Nadel (7) durch diese durchbrochen ist;
- wobei die zweiten Haltemittel einen Abstützbereich (61) aufweisen, gegen den das proximale Ende des Körpers der Spritze (5) abgestützt werden soll, wobei die Distanz zwischen der distalen Querwand (20) und dem Abstützbereich (61) so ist, dass während der Verwendung das Anschlussstück (8) zur Verbindung der Nadel (7) mit dem Körper der Spritze (5) in Abstützung gegen die distale Querwand (20) gehalten wird, wenn der Körper der Spritze (5) sich gegen den Abstützbereich (61) abstützt,
wobei der Körper (15) durch eine halbe Röhre gebildet wird,
wobei das Loch (22), das zum Durchgang der Nadel (7) in der distalen Querwand (20) enthalten ist, sich über zumindest eine Spalte (25) zur Außenseite dieser Wand öffnet, wobei die Spalte (25) ein Einführen der Nadel (7) in das Loch (22) in Querrichtung ermöglicht, und
wobei zwei im Wesentlichen parallele proximale Querwände (30, 31) vorhanden sind, die in Längsrichtung gegeneinander versetzt sind und die zwischen sich einen Aufnahmeraum (33) zur Aufnahme eines gegebenenfalls an dem Körper (5) der Spritze (2) vorhandenen proximalen Flansches (10) oder proximaler Querlaschen begrenzen, **dadurch gekennzeichnet, dass** der Aufnahmeraum (33) sich in Querrichtung im Wesentlichen in der gleichen Richtung öffnet wie die in der das Loch (22) zum Durchgang der Nadel (7) durch die Spalte (25) mit der Außenseite des Zusatzteils (1) in Verbindung steht.

2. Zubehörteil (1) nach Anspruch 1, **dadurch gekennzeichnet, dass** es zumindest ein Mittel (26) aufweist, das es ermöglicht, ein Blockieren der Rotation des Anschlussstücks (8) bezogen auf den Körper (15) des Zusatzteils (1) sicher zu stellen.

3. Zubehörteil (1) nach Anspruch 2, **dadurch gekennzeichnet, dass** das Mittel zum Blockierten in Form zumindest einer Zacke (26) ausgebildet ist, die über die distale Querwand (20) und/oder in einen an diese angrenzenden Rand (21) übersteht.

4. Zubehörteil (1) nach Anspruch 3, **dadurch gekennzeichnet, dass** es eine Vielzahl von Zacken (26) aufweist und dass die Zacken (26) im Wesentlichen in einer auf das Loch (22) bezogen radialen Richtung um das Loch (22) angeordnet sind, das in der distalen Querwand (20) gebildet ist, um den Durchgang der Nadel (7) zu ermöglichen.

5. Zubehörteil (1) nach einem der Ansprüche 1 bis 4, **dadurch gekennzeichnet, dass** das Anschlussstück (8) zur Verbindung der Nadel (7) mit dem Körper der Spritze (5) eine mehr oder weniger konische oder zylindrokonische Form aufweist, und dazu vorgesehen ist, in dem Loch (22) in der distalen Querwand (20) unter Verklemmen aufgenommen zu werden.

6. Zubehörteil (1) nach einem der Ansprüche 1 bis 5, **dadurch gekennzeichnet, dass** dieses als ein einzelnes Stück insbesondere durch Gießen einer synthetischen Masse hergestellt ist.

7. Zubehörteil (1) nach einem der Ansprüche 1 bis 6, **dadurch gekennzeichnet, dass** sein Körper (15) in zwei Teilen (16, 17) ausgeführt ist, von denen eines die Querwand (20) und das andere den Abstützbereich (61) aufweist, wobei die beiden Teile (16, 17) untereinander durch einen elastischen Bereich (18) verbunden sind, der in Längsrichtung des Zubehörteils (1) ausziehbar ist.

8. Zubehörteil (1) nach Anspruch 7, **dadurch gekennzeichnet, dass** der elastische Bereich (18) zumindest einen gekrümmten, durchbrochenen, gewellten oder schraubenförmigen Abschnitt (40) aufweist, der die beiden Teile (16, 17) des Körpers (15) des Zubehörteils (1) verbindet.

9. Zubehörteil (1) nach einem der Ansprüche 1 bis 8, **dadurch gekennzeichnet, dass** der Abstützbereich (61) dazu ausgebildet ist, einen Anschlag zu bilden, der das Verschieben der Kolbenstange (6) zulässt, sich aber entlang des Rückzugswegs des Kolbens der Spritze (2) oder eines Teils der Kolbenstange befindet.

10. Zubehörteil (1) nach einem der Ansprüche 1 bis 9, **dadurch gekennzeichnet, dass** der Abstützbereich (61) durch zumindest eine proximale Querwand (31) begrenzt ist, die diesen aufweist.

11. Zubehörteil (1) nach Anspruch 1, **dadurch gekennzeichnet, dass** der Aufnahmeraum (33) an den proximalen Flansch (10) oder die proximalen Querlaschen angepasst ist.

12. Zubehörteil (1) nach einem der Ansprüche 1 bis 11, **dadurch gekennzeichnet, dass** es Mittel zum Verrasten der Spritze darin aufweist.

13. Zubehörteil (1) nach einem der Ansprüche 1 bis 12, **dadurch gekennzeichnet, dass** es eine Verbindungswand (60) aufweist, die im Bereich der Fläche, die der Querwand (20) zugewandt ist, die dazu vorgesehen ist, das Anschlussstück (8) der Nadel (7) aufzunehmen, Querflächen (35) auf beiden Seiten des Körpers (15) des Zubehörteils (1) bildet, wobei die Querflächen (35) dazu vorgesehen sind, die Finger des Anwenders aufzunehmen und zu diesem Zweck in ergonomischer Weise ausgebildet sind.

14. Zubehörteil (1) nach einem der Ansprüche 1 bis 13, **dadurch gekennzeichnet, dass** sein Körper (15) zwei Einfassungen in Längsrichtung (19) aufweist, die den Aufnahmeraum für den Körper der Spritze (5) begrenzen, wobei die Einfassungen (19) Formen aufweisen, die sich in Richtung ihrer freien Ränder verjüngen.

15. Zubehörteil (1) nach einem der Ansprüche 1 bis 14, **dadurch gekennzeichnet, dass** es für einen Inhalt vom Typ "carpule" oder "cartridge" ausgebildet ist.
